# EUROPEAN PATENT APPLICATION

(11) **EP 3 950 054 A1**
(43) Date of publication of application: **09.02.2022**
(21) Application number: 19921410.7
(22) Date of filing: 04.11.2019
(51) Int. Cl.: A61N 5/06, A61N 5/067

(54) **OPTICAL GUIDE FOR RHINITIS TREATMENT DEVICE**

(30) Priority: 25.03.2019 KR 20190033953
(71) Applicant: Mirint Co., Ltd, Seongnam-si, Gyeonggi-do 13174 (KR)
(72) Inventor: KIM, Yong Ho, Seoul 05072 (KR)
(74) Representative: Viering, Jentschura & Partner mbB Patent- und Rechtsanwälte
(86) International application number: PCT/KR2019/014783
(87) International publication number: WO 2020/197027

(57) **Abstract**

The present invention relates to optical guides for a rhinitis treatment device, and the purpose of the present invention is to prevent partial breaking and incomplete manufacturing by performing manufacturing through injection molding, thereby preventing imbalance and the like in quality. Particularly, the present invention relates to optical guides for a rhinitis treatment device, the optical guides being manufactured through injection molding so as to minimize, even without including a cladding, light leakage that can be generated on the end side thereof. In addition, inducing diffusion in a limited range, which is required for treatment, can minimize the battery consumption of a device and, simultaneously, ensure treatment efficiency according to the diffusion.

## Description

### [Technical Field]

The present invention relates to optical guides for a rhinitis treatment device that are machined through injection molding, thereby preventing partial breaking and imperfect machining from occurring to thus avoid an imbalance in the quality thereof.

In specific, the present invention relates to optical guides for a rhinitis treatment device that are machined through injection molding, thereby minimizing the light leakage occurring on the sides of ends thereof, even while having no cladding.

Further, the present invention relates to optical guides for a rhinitis treatment device that can induce light diffusion within a restricted range, thereby minimizing the consumption of battery power of the device and ensuring an effective treatment.

### [Background Art]

Generally, there is a high probability that contemporary people may be infected by cold, rhinitis, and respiratory diseases due to temperature differences generated by the change of seasons or the increase in environmental pollutants and air pollutants.

In specific, children may easily suffer from diseases because they have weak resistance to the diseases, and further, they may have the lack of ability to adjust their body temperature by themselves, so that they may be frequently inflected by viral diseases such as cold, rhinitis, and respiratory diseases.

If a person has a cold, he or she has a runny or stuffy nose, and particularly, in the case of children, respiratory troubles may occur to cause dangerous situations. Even in the case of adults, it is hard for them to have normal respiration, thereby causing problems in their activities and having a high probability that the cold may be developed to diseases such as rhinitis, sinusitis, and the like.

Also, rhinitis causes an infection on the mucosa of the nasal cavity in the interior of the nose as the respiratory organ of the human body, and the most common kinds of rhinitis are viral and allergic rhinitis. If a person is infected by rhinitis, it is hard that he or she breathes normally, which causes headache, lack of attention, and the like.

A rhinitis treatment device used to release the symptoms of rhinitis or treat the rhinitis is typically classified into a device using ultrasonic spraying that transforms a treatment medicine such as saline, benzethonium chloride, alcohol, and the like into steam with fine particles by means of application of ultrasonic waves and a device using compression air spraying that makes use of the vapor generated by heating the treatment medicine or saline to a given temperature and expands and sprays the vapor.

Among them, the device using compression air spraying can spray the treatment medicine finely and strongly by means of air pressure, and when compared with the device using ultrasonic spraying, the device using compression air spraying can provide a better treatment, so that the device using compression air spraying is widely used.

The rhinitis treatment devices using ultrasonic spraying and compression air spraying may cause excessive consumption amounts of treatment medicine whenever they are used, and as they directly spray the liquid medicine as a foreign substance into the affected region of the nose, further, the impact generated upon the spraying may give stimulations to the nose to thus cause other diseases related to rhinitis, thereby making a patient feel uncomfortable in the use thereof. In specific, the children or elder who may be easily infected by rhinitis are likely to avoid the use of the conventional rhinitis treatment devices, and as the rhinitis treatment devices are used in a state of being placed on given spaces, many limitations in their use may occur.

Recently, a laser rhinitis treatment device has been developed to radiate light of a semiconductor laser to the nasal cavity through low level laser therapy (LLLT) and thus improve the symptoms of allergic rhinitis through biostimulation effects, but because of the straightness of the light of the laser, the treatment range of the rhinitis may be restricted so that the treatment effectiveness may decrease.

So as to solve such problems, accordingly, optical guides for a rhinitis treatment device, which have been suggested by the same applicant as the invention, have ends grinded slantly to induce the diffusion of light. However, in this case, it is hard that the conventional optical guides can ensure the consistence in radiating directions and cause problems in the quality thereof such as irregularity of light output within the treatment range, and the like.

### [Disclosure]

### [Technical Problem]

Accordingly, the present invention has been made to solve the above-mentioned problems, and it is an object of the present invention to provide optical guides for a rhinitis treatment device that are machined through injection molding, thereby preventing partial breaking and imperfect machining from occurring.

It is another object of the present invention to provide optical guides for a rhinitis treatment device that are machined through injection molding, thereby minimizing the light leakage occurring on the sides of ends thereof, even while having no cladding.

It is yet another object of the present invention to provide optical guides for a rhinitis treatment device that are configured to allow a portion of each end to be horizontally formed and to allow the other portion thereof to be slantly formed, so that the diffusion of laser light can be induced to a restricted range needed for a rhinitis treatment, thereby minimizing the consumption of battery power of the device and ensuring an effective treatment.

### [Technical Solution]

To accomplish the above-mentioned objects, according to the present invention, there are provided optical guides for a rhinitis treatment device that are inserted into the nose, while being protected by probes, to allow laser light to be incident thereon to radiate the laser light incident thereon to the interior of the nose, each optical guide including: a front portion formed to have a portion of one end formed horizontally; a slant portion formed to have the other portion of one end slanted downwardly; a bottom portion formed along the peripheral surface thereof from one end of the front portion to the lowermost point of the slant portion except the slant portion; and a peripheral portion formed along the peripheral surface thereof under the slant portion and the bottom portion.

Further, the front portion has an area of 1/3.1 to 1/3 of the diameter of the optical guide.

Further, the ratio of the maximum length of the front portion to the height of the bottom portion is 1: 3.6 to 1: 3.75.

Further, the slant portion has an area of 1/1.5 to 1/1.45 of the diameter of the optical guide.

Further, the ratio of the height of the bottom portion to the base length of the slant portion is 1: 1.7 to 1: 1.8.

Further, the front portion and the slant portion have an area of 1/1.8 to 1/2.2 of the diameter of the optical guide.

Further, the slant portion has an internal angle up to the horizontal axis of the uppermost point of the peripheral portion in the range of 55 to 62°.

Further, at least one or more surfaces of the slant portion and the peripheral portion have light-shielding layers adapted to prevent light leakage from occurring.

Further, the optical guides are machined through injection molding.

Further, the optical guides have no cladding on the outer peripheral surfaces thereof.

### [Effective Advantages of the Invention]

According to the present invention, the optical guides for a rhinitis treatment device are machined through injection molding, thereby preventing partial breaking and imperfect machining from occurring to thus avoid an imbalance in the quality thereof.

In specific, the optical guides for a rhinitis treatment device according to the present invention are configured to allow a portion of each end to be horizontally formed and to allow the other portion thereof to be slantly formed, so that the diffusion of laser light can be induced to a restricted range needed for a rhinitis treatment, thereby minimizing the consumption of battery power of the device and ensuring an effective treatment.

Accordingly, the optical guides for a rhinitis treatment device according to the present invention are machined to a specific shape through injection molding, thereby preventing the light leakage that may be caused because of no cladding from occurring and inducing uniform diffusion of light to a portion needed for the rhinitis treatment to thus enhance the treatment effectiveness.

### [Description of Drawings]

FIG. 1 is a perspective view showing a rhinitis treatment device with optical guides for a rhinitis treatment device according to the present invention.
FIG. 2 is a side view showing the optical guides for a rhinitis treatment device according to the present invention, when viewed on the left side of FIG. 1.
FIG. 3 is a top view showing each optical guide for a rhinitis treatment device according to the present invention when viewed on top side of FIG. 1.
FIGs. 4a to 4c are photographs showing the light radiated from each optical guide for a rhinitis treatment device according to the present invention.
FIG. 5 is a schematic side view showing each optical guide for a rhinitis treatment device according to the present invention, on which light-shielding layers are formed.
FIG. 6 shows comparison results for uniform output through laser light output power measurements according to shapes of the optical guides of the present invention.

### [Best Mode for Invention]

Optical guides for a rhinitis treatment device according to the present invention may have various exemplary embodiments, and hereinafter, the most preferred embodiment of the optical guides for a rhinitis treatment device according to the present invention will be explained with reference to the attached drawings.

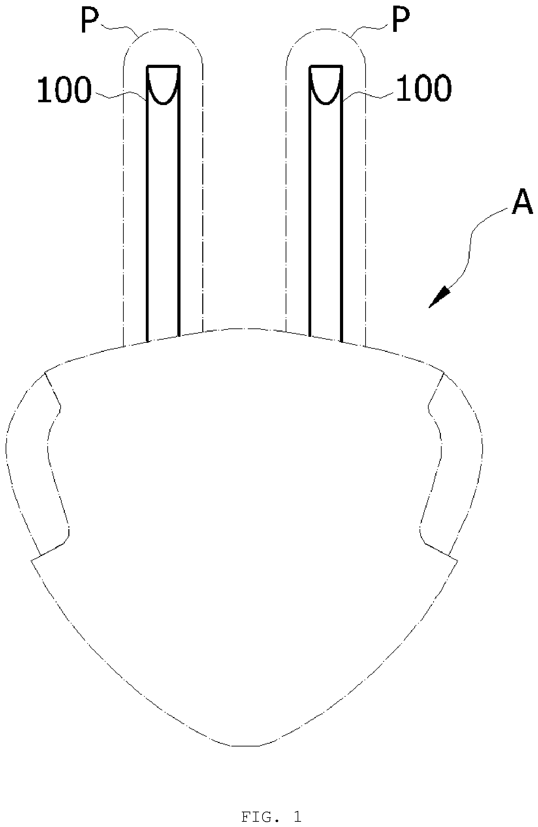

FIG. 1 is a perspective view showing a rhinitis treatment device with optical guides for a rhinitis treatment device according to the present invention.

Referring to FIG. 1, optical guides 100 for a rhinitis treatment device according to the present invention, which are provided for the rhinitis treatment device A that radiates light emitted from a laser to the nasal cavity to improve allergic rhinitis through biostimulation effects, are located inside probes P adapted to be inserted into the nasal cavity of a user to which a rhinitis treatment is applied.

Generally, the optical guides for a rhinitis treatment device have vertically cut ends or semi-circular ends, and accordingly, the light emitted from the laser moves very straightly from the ends of the optical guides or is diffused unnecessarily in every direction, thereby making treatment effectiveness deteriorated.

To solve such a problem, new optical guides having ends grinded diagonally have been suggested by the same applicant as the invention, but in this case, light is radiated only to a given direction. In specific, partial breaking and imperfect machining occurring during the machining for the ends of the optical guides may cause the light to be damaged, and accordingly, the diffusion angle of the light may be not regular.

Therefore, the optical guides 100 for a rhinitis treatment device according to the present invention are provided to prevent the laser light from being excessively diffused in the nasal cavity or being collected only to a given portion, and in specific, they are machined through injection molding to solve the problems occurring when they are grinded.

However, the optical guides 100 machined through the injection molding do not have any cladding process on the outer surface thereof, and in this case, light may leak from the sides of the ends of the optical guides 100.

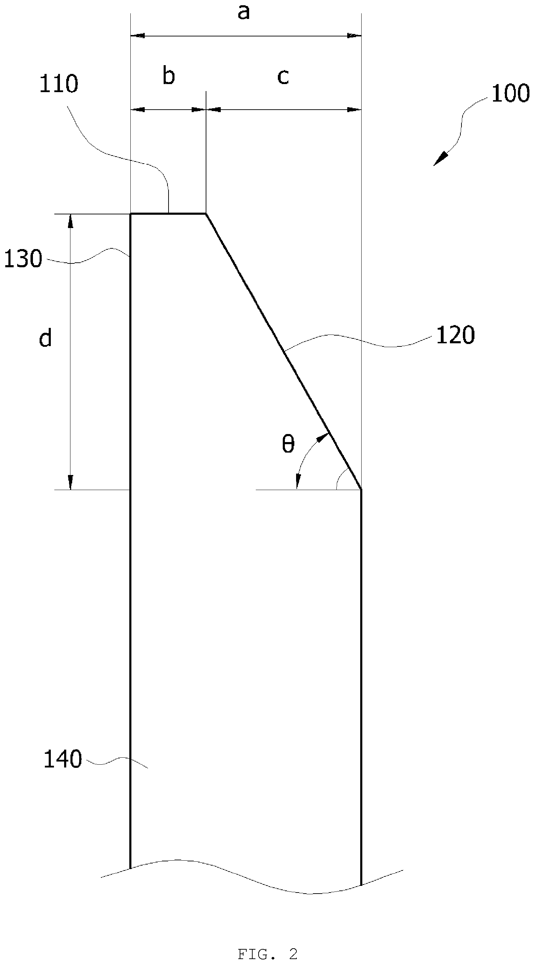

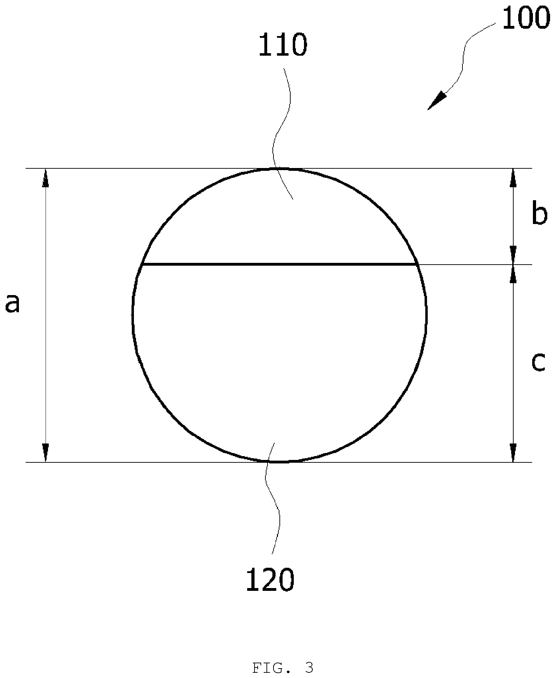

The present invention relates to the optical guides 100 for a rhinitis treatment device that are capable of preventing a treatment from being ineffective due to the unnecessary diffusion or collection of the laser light and achieving many conveniences in manufacturing and high uniformity in quality, and now, an explanation of the optical guides 100 for a rhinitis treatment device will be given in detail with reference to FIGs. 2 and 3.

FIG. 2 is a side view showing the optical guides for a rhinitis treatment device according to the present invention, when viewed on the left side of FIG. 1.

FIG. 3 is a top view showing each optical guide for a rhinitis treatment device according to the present invention when viewed on top side of FIG. 1.

Referring to FIG. 2, each optical guide 100 for a rhinitis treatment device according to the present invention is made through injection molding so that a portion of one end thereof is formed horizontally and the other portion thereof is slanted diagonally.

That is, each optical guide 100 according to the present invention has a front portion 110 formed to have a portion of one end thereof formed horizontally and a slant portion 120 formed to have the other portion of one end slanted.

On the entire cylindrical optical guide 100, further, the side peripheral surface of the optical guide 100 formed under the lowermost point of the slant portion 120 of the side peripheral surface is defined as a peripheral portion 140, and the side peripheral surface formed above the lowermost point of the slant portion 120 as a bottom portion 130.

That is, each optical guide 100 according to the present invention includes the bottom portion 130 forming the peripheral surface from the front portion 110 to the opposite position to the lowermost point of the slant portion 120 except the slant portion 120 and the peripheral portion 140 forming the peripheral surface under the bottom portion 130.

The bottom portion 130 faces the bottom surface of the nasal cavity located on the maxillary bone within the nasal cavity.

If it is desired to treat rhinitis through a laser, it is desirable that biostimulation is collected to the mucosa of the nasal cavity and the nasal turbinate. In this case, superoxide dismutase or catalase is activated to suppress reactive oxygen groups in blood, thereby most efficiently releasing the symptoms of the rhinitis generated due to hypersensitivity reaction.

Accordingly, it is desirable that the laser light incident onto each optical guide 100 according to the present invention is totally reflected onto the slant portion 120, while the laser light is being uniformly emitted only to the front portion 110 and the bottom portion 130. The laser light radiated through the front portion 110 and the bottom portion 130 is transferred to the mucosal cells of the nasal cavity, and in specific, the laser light radiated through the side periphery of the bottom portion 130 is transferred to the nasal turbinate.

However, each optical guide 100 according to the present invention may cause the laser light emitted through the front portion 110 and the bottom portion 130 to become irregularly radiated according to the angle of the slant portion 120.

The slant portion 120 of each optical guide 100 according to the present invention desirably has an internal angle θ (hereinafter, referred to as "inclination") up to the horizontal axis of the uppermost point of the peripheral portion 140 in the range of 55 to 62°.

To do this, as shown in FIGs. 2 and 3, the front portion 110 of each optical guide 100 according to the present invention is desirably formed to have an area of 1/3.1 to 1/3 of the diameter a of the optical guide 100.

In this case, the area becomes a maximum horizontal length or area b as shown in FIG. 2 or becomes a maximum vertical length or area b as shown in FIG. 3.

Further, the ratio of the maximum length b of the front portion 110 to the height d of the bottom portion 130 desirably is 1: 3.6 to 1: 3.75.

Accordingly, each optical guide 100 according to the present invention can provide the slant portion 120 with a desirable inclination θ obtained by calculating the maximum length b of the front portion 110 according to the diameter a thereof and the height d of the bottom portion 130 according to the maximum length b of the front portion 110.

Further, each optical guide 100 according to the present invention can provide the slant portion 120 with a desirable inclination θ with respect to the slant portion 120.

The slant portion 120 is slanted downward from one end of the front portion 110 up to one end of the peripheral portion 140, when viewed in FIG. 2.

As shown in FIGs. 2 and 3, the slant portion 120 is desirably formed to have an area of 1/1.5 to 1/1.45 of the diameter a of the optical guide 100.

In this case, the area becomes a base length or area c as shown in FIG. 2 or becomes a maximum vertical length or area c as shown in FIG. 3.

Further, the ratio of the height d of the bottom portion 130 to the base length c of the slant portion 120 desirably is 1: 1.7 to 1: 1.8.

Accordingly, each optical guide 100 according to the present invention can provide the slant portion 120 with a desirable inclination θ obtained by calculating the base length c of the slant portion 120 according to the diameter a thereof and the height d of the bottom portion 130 according to the base length c of the slant portion 120.

The front portion 110 and the slant portion 120 are formed to have an area of 1/1.8 to 1/2.2 of the diameter a of the optical guide 100.

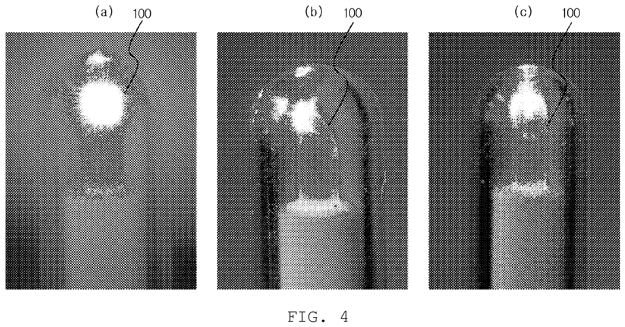

FIGs. 4a to 4c are photographs showing the light irradiated from the optical guides for a rhinitis treatment device according to the present invention.

Through the front portion 110 horizontally formed and the slant portion 120 slantly formed of each optical guide 100 according to the present invention, as shown in FIGs. 4a to 4c, laser light with uniform output power can be radiated from the front portion 110 and the bottom portion 130. Further, the total reflection is performed onto the slant portion 120, thereby desirably having no laser light radiation thereonto.

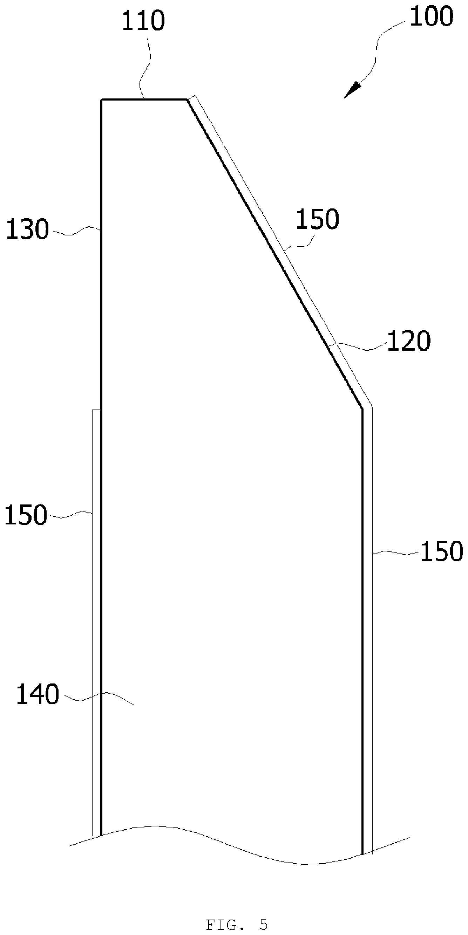

FIG. 5 is a schematic side view showing each optical guide for a rhinitis treatment device according to the present invention, on which light-shielding layers are formed.

Referring to FIG. 5, each optical guide 100 according to the present invention includes light-shielding layers 150 for preventing the laser light from leaking to one side or entire surface of the peripheral portion 140, thereby avoiding the light leakage occurring when the injection molding is performed, without any cladding process.

As shown in FIG. 5, the light-shielding layers 150 may be formed on at least one or more surfaces of the slant portion 120 and the peripheral portion 140.

In specific, the light-shielding layer 150 formed on the peripheral portion 140 is desirably formed up to the lowermost point of the bottom portion 130.

As a result, the light leakage from other portions except the front portion 110 and the bottom portion 130 can be reliably prevented.

Hereinafter, test results obtained by applying embodiments of the present invention will be explained in detail.

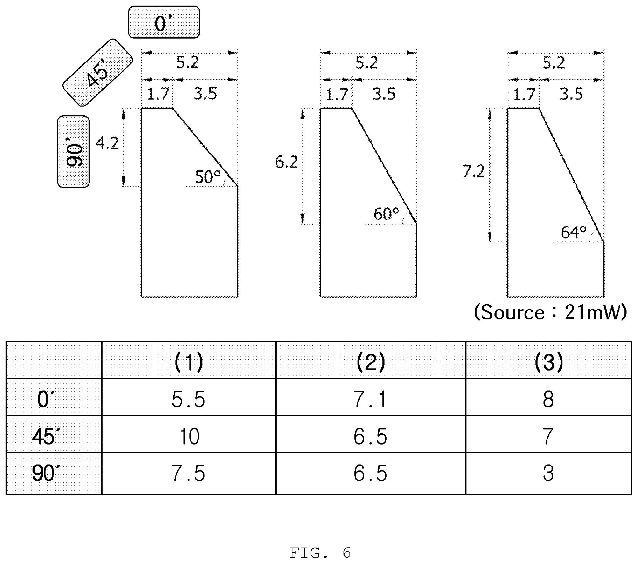

FIG. 6 shows comparison results for uniform output through laser light output power measurements according to shapes of the optical guides of the present invention, and in this case, the laser light output power of the front portion 110 and the bottom portion 130 is measured according to the lengths of the front portion 110, the slant portion 120, and the bottom portion 130.

In the case of Comparison example 1 wherein the diameter of the optical guide is 5.2 mm, the maximum length of the front portion is 1.7 mm, the base length of the slant portion is 3.5 mm, and the height of the bottom portion is 4.2 mm to thus have the inclination of 50° of the slant portion, in the case of Example 1 of the present invention wherein the diameter of the optical guide is 5.2 mm, the maximum length of the front portion is 1.7 mm, the base length of the slant portion is 3.5 mm, and the height of the bottom portion is 6.2 mm to thus have the inclination of 60° of the slant portion, in the case of Comparison example 2 wherein the diameter of the optical guide is 5.2 mm, the maximum length of the front portion is 1.7 mm, the base length of the slant portion is 3.5 mm, and the height of the bottom portion is 7.2 mm to thus have the inclination of 64° of the slant portion, comparison results for uniform output are obtained.

Referring to Comparison example 1 of FIG. 6, the laser output value measured at an angle of 0° was 5.5 mW, the laser output value measured at an angle of 45° was 10 mW, and the laser output value measured at an angle of 90° was 7.5 mW.

Referring to Example 1, contrarily, the laser output value measured at an angle of 0° was 7.1 mW, the laser output value measured at an angle of 45° was 6.5 mW, and the laser output value measured at an angle of 90° was 6.5 mW, and referring to Comparison example 2, the laser output value measured at an angle of 0° was 8 mW, the laser output value measured at an angle of 45° was 7 mW, and the laser output value measured at an angle of 90° was 3 mW.

In the case of Comparison example 1 wherein the inclination of the slant portion was 50°, the maximum laser light output power was the highest power, but the laser light output power was more collected to 90° when compared to 0° and 45°. In the case of Comparison example 2 wherein the inclination of the slant portion was 64°, further, the laser light output power was more collected to 0° when compared to 45° and 90°.

In the case of Example 1 wherein the inclination of the slant portion was 60°, contrarily, the deviation in the laser light output power measured at 0°, 45°, and 90° was substantially low, so that it could be checked that the laser light was uniformly outputted.

It can be therefore appreciated that the lower the inclination of the slant portion 120 is, the more the laser light is collected to the bottom portion 130, and the higher the inclination of the slant portion 120 is, the more the laser light is collected to the front portion 110.

Accordingly, it is desirable that each optical guide 100 according to the present invention has the slant portion 120 with the inclination of 60°. To do this, desirably, the maximum length b of the front portion 110 is 1/3.03 of the diameter a of the optical guide 100 and the height d of the bottom portion 130 is 3.7 times of the maximum length b of the front portion 110. In the case of the slant portion 120, further, it is desirable that the base length c of the slant portion 120 is 1/1.48 of the diameter a of the optical guide 100 and the height d of the bottom portion 130 is 1.78 times of the base length c of the slant portion 120.

The optical guides for a rhinitis treatment device according to the present invention are configured to allow a portion of one end to be horizontally formed and to allow the other portion thereof to be slanted diagonally, so that the diffusion of the laser light is induced to a restricted range needed for the rhinitis treatment, thereby minimizing the consumption of battery power of the device, and further, the optical guides for a rhinitis treatment device according to the present invention are configured to prevent the light leakage that may occur due to no cladding, thereby inducing uniform diffusion of the laser light to the portion needed for the rhinitis treatment to ensure an effective treatment.

Up to now, the optical guides for a rhinitis treatment device according to the present invention have been explained with reference to the particular illustrative embodiments, but it is not to be restricted by the embodiments but only by the appended claims. It is to be appreciated that those skilled in the art can change or modify the embodiments without departing from the scope and spirit of the present invention.

Therefore, it should be understood that the embodiments of the invention are just exemplary, without any restriction.

### [Explanations of Reference Numerals]

- 100:: Optical guides for a rhinitis treatment device
- 110:: Front portion
- 120:: Slant portion
- 130:: Bottom portion
- 140:: Peripheral portion
- 150:: Light-shielding layer

### [Industrial Applicability]

The present invention can be applied to medical and wearable healthcare fields and the fields similar or related thereto, thereby improving the reliability and competitiveness of products.

## Claims

1. Optical guides for a rhinitis treatment device, configured to be inserted into the nose, while being protected by probes, to allow laser light to be incident thereon to radiate the laser light incident thereon to the interior of the nose, each optical guide comprising:
a front portion formed to have a portion of one end formed horizontally;
a slant portion formed to have the other portion of one end slanted downwardly;
a bottom portion formed along the peripheral surface thereof from one end of the front portion to the lowermost point of the slant portion except the slant portion; and
a peripheral portion formed along the peripheral surface thereof under the slant portion and the bottom portion.

2. The optical guides according to claim 1, wherein the front portion has an area of 1/3.1 to 1/3 of the diameter of the optical guide.

3. The optical guides according to claim 1, wherein the ratio of the maximum length of the front portion to the height of the bottom portion is 1: 3.6 to 1: 3.75.

4. The optical guides according to claim 1, wherein the slant portion has an area of 1/1.5 to 1/1.45 of the diameter of the optical guide.

5. The optical guides according to claim 1, wherein the ratio of the height of the bottom portion to the base length of the slant portion is 1: 1.7 to 1: 1.8.

6. The optical guides according to claim 1, wherein the front portion and the slant portion have an area of 1/1.8 to 1/2.2 of the diameter of the optical guide.

7. The optical guides according to claim 1, wherein the slant portion has an internal angle up to the horizontal axis of the uppermost point of the peripheral portion in the range of 55 to 62°.

8. The optical guides according to claim 1, wherein at least one or more surfaces of the slant portion and the peripheral portion have light-shielding layers adapted to prevent light leakage from occurring.

9. The optical guides according to claim 1, machined through injection molding.

10. The optical guides according to claim 1, having no cladding on the outer peripheral surfaces thereof.
